# EUROPEAN PATENT APPLICATION

(11) **EP 2 664 587 A2**
(43) Date of publication of application: **20.11.2013**
(21) Application number: 11855618.2
(22) Date of filing: 28.12.2011
(51) Int. Cl.: C02F 3/34, C12N 1/20, C02F 103/24, C02F 103/20

(54) **BIOLOGICAL TREATMENT METHOD AND WASTE-WATER TREATMENT AGENT FOR REFRACTORY WASTEWATER**

(30) Priority: 14.01.2011 KR 20110004159; 25.11.2011 KR 20110124238
(71) Applicant: Korea Maritime University Industry-Academic Cooperation Foundation, Busan 606-080 (KR); BM Co., Ltd, Busan 604-848 (KR)
(72) Inventor: KIM, Hong-gi, Changwon-si Gyeongsangnam-do 645-520 (KR); KIM, Jong-tae, Daejeon 302-874 (KR); KOH, Sung-cheol, Busan 613-756 (KR); KIM, In-soo, Busan 612-050 (KR)
(74) Representative: Ferrari, Barbara
(86) International application number: PCT/KR2011/010201
(87) International publication number: WO 2012/096462

(57) **Abstract**

The present invention relates to a biological treatment method for sewage and wastewater, tannery wastewater and livestock manure, and, more particularly, relates to a biological treatment method and a waste-water treatment agent for refractory wastewater, whereby not only is it possible to treat refractory wastewater biologically without any physical/chemical pre-processing but it is also possible to reduce the occurrence of the odors and the sludge which occur in the process of wastewater treatment. The biological treatment method for refractory wastewater of the present invention comprises: a step of producing a complex microbial liquid by maintaining, at between 15 and 28°C, a complex microbial liquid obtained by mixing between 0.01 and 1 percent by weight of mixed microorganisms BM-S-1 (Repository Deposit No. KCTC 11789BP), between 0.1 and 1 percent by weight of powdered chaff, between 0.1 and 1 percent by weight of powdered peat moss, between 1 and 5 percent by weight of molasses, between 0.01 and 1 percent by weight of shiitake mushroom waste wood dust and between 92 and 98 percent by weight of water; a mixed stock production step entailing the mixing of from 50 to 95 percent by weight of a culture stock with from 5 to 50 percent by weight of the complex microbial liquid; a high-temperature inoculation step in which from 0.01 to 1 part by weight of mixed microorganisms (BM-S-1) is inoculated into 100 parts by weight of the mixed stock, under high-temperature conditions of between 65°C to 85°C; a culturing step involving culturing of the mixed stock that has undergone the high-temperature inoculation; a drying step in which the cultured mixed stock is dried; a microbial starting broth production step in which the dried mixed stock is cultured in the liquid state; and a microbe activation step in which the microbe population is multiplied in order to introduce the microbial starting broth, that has undergone the microbial starting broth production step, into wastewater.

## Description

### Technical Field

The present invention relates to a method for biologically treating sewage, wastewater, tannery wastewater and livestock manure, and more particularly to a method for biologically treating refractory wastewater, which can biologically treat refractory wastewater without performing physical or chemical pretreatment and can also reduce the generation of various offensive odors and sludge in a wastewater treatment process, and to an agent for treating wastewater.

### Background Art

Generally, high-concentration refractory wastewater which is discharged from industries is responsible for the contamination of water in various aquatic ecological environments. Among industrial sources that discharge high-concentration refractory wastewater, livestock farms are mostly petty and stand close together in water supply source areas adjacent to large aquatic ecological environments, and thus water contamination caused thereby is significantly serious. In addition, in livestock farms, a large amount of sludge (which is formed by precipitation of impurities contained in water or oil) is generated during the treatment of livestock manure, and the generated sludge is treated at high costs and becomes a serious problem in the management of livestock farms.

Moreover, tannery wastewater which is generated in leather manufacturing companies is typical high-concentration refractory wastewater containing various organic materials, chemicals, heavy metals and the like and is treated by physical and chemical methods worldwide, and biological methods for treating tannery wastewater are used as auxiliary methods.

In Korea, in the case of tannery wastewater, the T-N discharge limit was 60 mg/l and the COD discharge limit was 90 mg/l. It was considered that these discharge limits were very strict limits which could not be substantially satisfied with current technologies. For this reason, in the year 2004, the Korean Ministry of Environment relaxed the T-N discharge limit to 200 mg/l for some raw hide processing facilities only. Even at present (August, 2010), leather manufacturing companies are requesting that the discharge limit be further relaxed.

Specifically, current technologies cannot treat such refractory wastewater at satisfactory levels. Furthermore, because biological treatment is insufficient for treatment of such wastewater, physical and chemical pretreatment is performed before biological treatment, and thus in most cases, sludge is caused by precipitation of large amounts of chemicals. In addition, a severe offensive odor occurs during the treatment process, and it is difficult to treat sludge generated during the treatment process. Accordingly, a fundament solution to these problems and an effective method for treatment of wastewater are been urgently required.

### Disclosure

### Technical Problem

Accordingly, the present invention has been made in order to solve the above-mentioned problems, and an object of the present invention is to propose a possibility of biologically treating high-concentration refractory wastewater such as livestock manure or tannery wastewater and to a method for biologically treating refractory wastewater, which can reduce the generation of sludge during wastewater treatment.

Another object of the present invention is to provide a wastewater treatment agent for treating high-concentration refractory wastewater such as livestock manure or tannery wastewater.

### Technical Solution

In order to accomplish the above objects, the present invention provides a method for biologically treating refractory wastewater, the method comprising the steps of: maintaining a mixture of 0.01-1 wt% of microorganism BM-S-1 (accession number: KCTC 11789BP), 0.1-1 wt% of powdery chaff, 0.1-1 wt% of powdery peat moss, 1-5 wt% of molasses, 0.01-1 wt% of shiitake mushroom waste wood powder and 92-98 wt% of water at 15 to 28 °C to prepare a liquid microbial agent; mixing 50-90 wt% of a medium with 5-50 wt% of the liquid microbial agent to prepare a mixed material; inoculating 100 parts by weight of the mixed material with 0.01-1 part by weight of the microbial mixture (BM-S-1) at a high temperature of 65 °C to 85 °C; incubating the mixed material inoculated at a high temperature; drying the incubated mixed material; incubating the dried mixed material in a liquid state to prepare a microbial solution; and proliferating and activating microorganisms of the microbial solution before introducing the microbial solution into wastewater.

According to a preferred embodiment of the present invention, the wastewater is selected from among livestock manure, tannery wastewater and refractory wastewater, and the microbial solution is fixed on a carbohydrate medium.

A wastewater treatment agent according to a preferred embodiment of the present invention is prepared by mixing 0.01-1 wt% of microorganism BM-S-1 (accession number: KCTC 11789BP), 0.1-1 wt% of powdery chaff, 0.1-1 wt% of powdery peat moss, 1-5 wt% of molasses, 0.01-1 wt% of shiitake mushroom waste wood powder and 92-98 wt% of water.

### Advantageous Effects

According to the present invention, the following effects can be provided.

According to the present invention, livestock manure and tannery wastewater, known as high-concentration refractory wastewater, are treated by the biological method only. The treatment efficiency of the biological method of the present invention is higher than that of a conventional physical/chemical process, and the method of the present invention significantly reduces the generation of offensive odors and the generation of sludge.

In addition, environmental improvement charges of farmhouses or enterprises can be reduced, thus reducing production costs.

### Description of Drawings

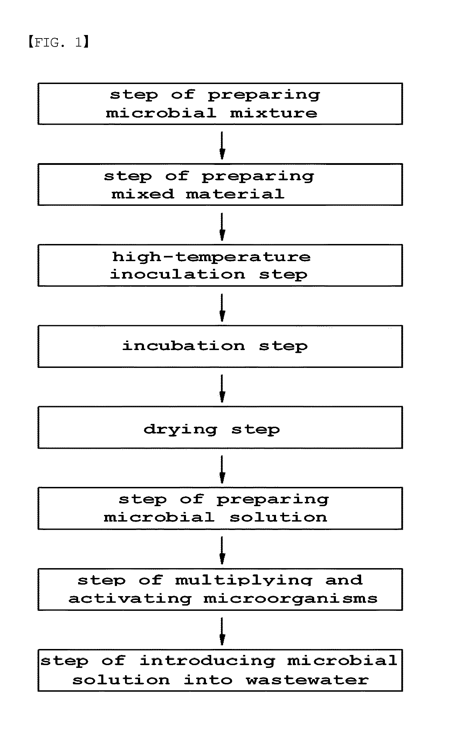

FIG. 1 is a flowchart showing the biological treatment of refractory wastewater according to a preferred embodiment of the present invention.

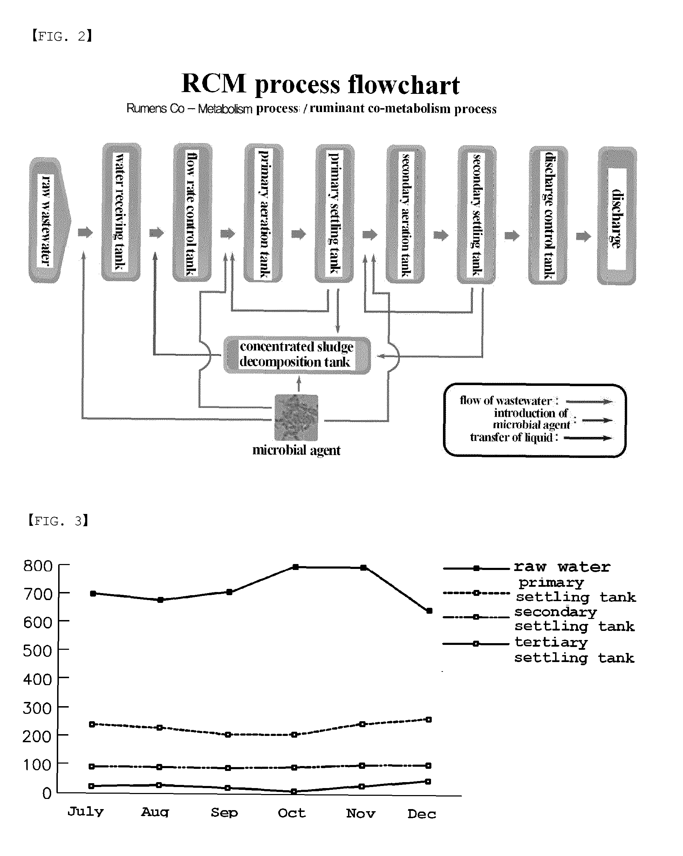

FIG. 2 shows a configuration for the biological treatment of refractory wastewater according to a preferred embodiment of the present invention.
FIG. 3 shows T-N measurement values obtained in Test Example 1 of the present invention.

### Best Mode

Hereinafter, the present invention will be described in further detail.

The present invention is directed to a biological treatment method for efficiently purifying tannery wastewater, livestock manure or the like, which has a high degree of contamination and contains large amounts of poorly degradable materials, unlike general river sewage.

A microbial mixture (BM-S-1) that is used in the present invention was deposited at the Korean Collection for Type Culture (KCTC) of the Korea Research Institute of Bioscience and Biotechnology under accession number KCTC 11789BP on October 20, 2010. The results of analysis of the microbial mixture (BM-S-1) showed that the microbial mixture is composed of about 130 bacterial strains (see Table 1), including Prevotellaceae_uc_s, *Lactobacillus_*uc, *Lactobacillus parabuchneri,* Lactobacillaceae_uc_s, *Lactobacillus paracasei, Lactobacillus parafarraginis, Lactobacillus camelliae, Lactobacillus manihotivorans, Acetobacter lovaniensis, Ethanoligenens_*uc, Veillonellaceae_uc_s, *Lactobacillus similis, Lactobacillus harbinensis* and Rhodospirillales_uc_s, and yeast *(Candida boidinii) .*

Table 1 below shows the distribution of species in the microbial mixture (BM-S-1), analyzed by pyrosequencing.

**Table 1**

| Name of species | Ratio (%) |
|---|---|
| Prevotellaceae_uc_s | 22.2 |
| *Lactobacillus_*uc | 17.7 |
| *Lactobacillus parabuchneri* | 6.9 |
| Lactobacillaceae_uc_s | 6.5 |
| *Lactobacillus paracasei* | 5.8 |
| *Lactobacillus parafarraginis* | 4.3 |
| *Lactobacillus camelliae* | 3.0 |
| *Lactobacillus manihotivorans* | 2.4 |
| *Acetobacter lovaniensis* | 2.3 |
| *Lactobacillus collinoides* | 2.2 |
| *Lactobacillus vini* | 2.0 |
| *Lactobacillus hilgardii* | 1.8 |
| *Lactobacillus pentosus* | 1.7 |
| *Lactobacillus rapi* | 1.5 |
| *Lactobacillus pantheris* | 1.3 |
| *Ethanoligenens_*uc | 1.2 |
| Veillonellaceae_uc_s | 1.2 |
| *Lactobacillus similis* | 1.2 |
| *Lactobacillus harbinensis* | 1.0 |
| Rhodospirillales_uc_s | 0.5 |
| Others | 13.8 |
| Total | 100.0 |

| | |
|---|---|
| *results of analysis of a total of 6801 clones;** uc_s means unclassified species. | |

*Lactobacillus* sp. inhibits the growth of harmful microorganisms such as pathogenic microorganisms by making the surrounding environment acidic or generating hydrogen peroxide. It lives mainly in waste plant matter and is also found in the bowels of humans or animals while showing probiotic activity. The Rhodospirillales order is divided into Acetobacteraceae and Rhodospirillaceae. Acetobacteraceae includes *Acetobacter lovaniensis* shown in Table 1, and *Acetobacter lovaniensis* is an aerobic microorganism that forms acetic acid from alcohol and lives in the root, stem, leaf and the like of various plants (sugar canes, sweet potatoes, coffee trees, tea plants, bananas, etc.).

Rhodospirillaceae includes purple non-sulfur bacteria and green non-sulfur bacteria, which either grow using various organic acids or ethanol, produced by *Lactobacillus* sp., *Acetobacter* sp. and other anaerobic bacteria *(Ethanoligenens* sp.), which are present in the microbial agent of the present invention, or fix CO₂ through a photosynthetic process and play a major role in the purification of wastewater contaminated with organic materials. Prevotellaceae which is present in the microbial agent of the present invention at a significant density is present in the bowels of normal warm-blooded mammals (humans, animals, etc.), and is believed to function to convert sugars to succinic acid or acetic acid and contribute to the rapid decomposition of organic materials.

It was found that the dominant yeast identified in the microbial agent of the present invention was *Candida boidinii,* which is believed to make physiologically active substances such as vitamins or amino acids, which contribute to the growth of the microbial mixture (BM-S-1) and the decomposition of organic matter.

This microbial mixture (BM-S-1) was isolated in the following manner.

A soil sample (bamboo humus, ruminant non-digested material, or broad-leaved tree humus) was collected and mixed with a culture medium (rice bran, chaff, sawdust, egg shells, other shells, or peat moss) crushed to a size of 80-120 mesh, and the mixture was adjusted to a water activity of 40-60% and cultured on soil in a semi-shade condition for 90 days.

A medium obtained by mixing 10 wt% of rice bran, 40 wt% of chaff, 25 wt% of peat moss and 25 wt% of sawdust was adjusted to a water activity of 60%, and then the above-cultured sample was inoculated onto the medium in an amount of 0.01 parts by weight based on 100 parts by weight of the medium and was fermented while shaking at a temperature of 80 to 90 °C for 4 hours, and after 3 weeks it was fermented, thereby preparing a powdery microbial mixture having a water concentration of 8% or less. The isolated microbial mixture (BM-S-1) that is used in the present invention was deposited at the Korean Collection for Type Culture (KCTC) of the Korea Research institute of Bioscience and Biotechnology under accession number KCTC 11789BP on October 20, 2010.

The method for biologically treating refractory wastewater using the isolated microbial mixture comprises the steps of preparing a liquid microbial agent, preparing a mixed material, inoculating the mixed material at a high temperature, incubating the mixed material, drying it, preparing a microbial solution, and activating microorganisms.

In the step of preparing the liquid microbial agent, a mixture of 0.01-1 wt% of microorganism BM-S-1 (accession number: KCTC 11789BP), 0.1-1 wt% of powdery chaff, 0.1-1 wt% of powdery peat moss, 1-5 wt% of molasses, 0.01-1 wt% of shiitake mushroom waste wood powder and 92-98 wt% of water is maintained at a temperature of 15 to 28 °C while air is introduced therein at a rate of 6 x 10³ to 8 × 10³ L/min for 2-4 (aeration process), and the aeration process is stopped for a period of 2-4 days. The aeration process and the non-aeration process are repeatedly performed for 18-36 days, thereby preparing the liquid microbial agent. The conditions of the aeration process and the like are the optimum results determined based on the results of studies conducted by the present inventors, and the above culture conditions can be somewhat modified without departing from the scope of the present invention.

After preparing the liquid microbial agent, 50-95 wt% of at least one medium selected from the group consisting of pre-fermented peat moss and chaff is mixed with 5-50 wt% of the liquid microbial agent to prepare a mixed material.

Then, the step of inoculating 0.01-1 part by weight of the microbial mixture (BM-S-1) onto 100 parts by weight of the mixed material is performed.

The microbial agent of the present invention comprises, as a medium, chaff or peat moss, which is relatively easily available and inexpensive. In addition, forest byproducts, agricultural byproducts or food waste may be used as the medium. As the medium, chaff, peat moss, forest byproducts and agricultural byproducts may be used alone or in combination, but the medium is preferably used in a powdery state after it is crushed to a size of about 80-160 mesh and pre-fermented before use.

The mixed and crushed materials are placed in a shaking incubator, and the water content is adjusted to provide an environment suitable for microbial fermentation, after which the microbial mixture (BM-S-1) is inoculated.

The microbial mixture can be prepared in a powdery state by collecting a microbial phase from soil having seasonal and environmental diversities without filtration, and adapting the collected microbial phase to the environment on a carbohydrate medium for 6-9 months to remove harmfulness from the microbial phase.

In the preparation of the microbial agent according to the present invention, the microbial mixture (BM-S-1) is preferably inoculated in an amount of 0.01-1 part by weight based on 100 parts by weight of the mixed material.

The step of inoculating the mixed material at a high temperature of 65 °C to 85 °C for 4-6 hours is performed. In view that the fact that common microbial inoculation is generally performed at a temperature ranging from 20 °C to 40 °C, it can be seen that the inoculation in the method of the present invention is performed at a high temperature. Particularly, in an embodiment of the present invention, the medium inoculated with the microbial mixture is stirred at a temperature of 65 °C to 85 °C for 4-6 hours at a speed of 60-180 rpm. The stirring is performed at 65 °C or higher in order to induce the thermal denaturation of the medium components to induce the proliferation of soil microorganisms, but is performed at 85 °C or lower in order to induce the activation of the microbial mixture according to the present invention.

The reason why the high-temperature inoculation is selected is because the diversity of strains in culture at a high temperature of 65 to 85 °C is 3-5 times higher than that in culture at a middle/low temperature of 20 to 40 °C.

After the high-temperature inoculation step, the cultured material is optionally naturally cooled to room temperature, and is then placed in a porous container and cultured for 28-45 days, followed by drying. The dried material may be ground to a size of 120 mesh or less to maximize water affinity, thereby preparing a microbial fine powder product having high water affinity. In addition, the method of the present invention may further comprise a step of adding 13-16 parts by weight of the liquid microbial agent to the post-fermented powder and molding the mixture using a molding device.

Although the drying or grinding process may also be performed to provide powder, the molding process may be performed if necessary, and the water is supplied for molding. The molding process is not specifically limited, but in an example of the present invention, the cultured microbial agent was molded using a molding machine having a treatment capacity of 500-700 kg per hour, thereby obtaining pellets.

After the culture or molding step, an aging step of post-fermenting the cultured microbial agent may also be performed. The drying step may be performed using any known drying method that does not cause thermal denaturation of microorganisms, and is not specifically limited. In an example of the present invention, hot-air drying at 40 to 60 °C was performed.

In addition, the method of the present invention comprises a step of culturing the dried material in a liquid state to prepare a microbial solution, and a step of proliferating and activating the microorganisms of the microbial solution before adding the microbial solution to wastewater.

This is because it is effective to add microorganisms activated *in situ* to a wastewater treatment tank so that the function of the microorganisms can be exhibited rapidly, compared to adding the microorganisms directly to the wastewater treatment tank. In order to activate the microorganisms as described above, it is more advantageous to induce the activation of the microorganisms by dilution to activate the microorganisms in a liquid medium containing 10-20 wt% of effluent wastewater to which the microbial solution is to be applied. In other words, the use of the activation medium is more preferable because it increases the activity of the microorganisms and shortens the degradation period.

The method of adding the microorganisms after activation is performed in order to stably and continuously maintain the activity of the microorganisms, and specific examples of this method are already known in the art.

According to the wastewater treatment method of the present invention, tannery wastewater or livestock manure, which have high COD values and contain high concentrations of nitrogen compounds, can be treated using the microbial agent of the present invention so that the values are reduced below standard limits. In addition, the microbial agent of the present invention may, if necessary, contain various additives, for example, minerals (flocculants), alginic acid and its salts, organic acids, a protective colloidal thickener, an agent that is used in molding, and the like. The microbial agent that is prepared as described above may be added using any method that can uniformly disperse the microbial agent in a treatment tank. For example, the microbial agent in a storage container may be manually added directly to wastewater in a treatment tank while air is introduced or stirring is performed using a stirrer. The total capacity of a plurality of wastewater treatment tanks and the retention time vary depending on the amount of wastewater, but the retention time of wastewater in the plurality of treatment tanks is generally adjusted to the range from about 0.3 days to about 28 days. Particularly, the retention time is preferably adjusted to a range of about 0.5 days to about 11 days. Also, the number of treatment tanks is not limited, but is preferably 3-5 from the viewpoint of efficiency and equipment cost.

Treatment with the microbial agent is controlled by measuring pH, dissolved oxygen (DO), the COD values before and after treatment, and the like. The pH is 4.0-8.5, and preferably 5.5-8.0, and a narrower pH range can be selected depending on the nature of the wastewater. The DO is 3.0 mg/l-13.0 mg/l, and preferably 5.0 mg/l-9.0 mg/l. The pH can be controlled by addition of an acid or alkali, and the DO can be controlled by adding air to sewage to control water drainage and purification. The concentration of a specific compound can be measured by direct quantification, but CODmn corresponding to concentration is actually used. The measurement of CODmn is preferably performed by measuring concentrations in both an inlet of a first wastewater treatment tank and an outlet of a final treatment tank. In the wastewater treatment process, a carbon source, a nitrogen source, an organic nutrient source or an inorganic salt, which is suitable for growth of microorganisms, can be introduced. Examples of the organic nutrient source include polypeptone, yeast extract, meat extract, molasses and the like, and examples of the inorganic nutrient source include various phosphates, magnesium salts and the like. The organic nutrient source is added in an amount of about 0.001-0.005 wt%, and preferably about 0.001-0.002 wt%, based on the weight of wastewater, and the inorganic nutrient source is added in an amount of about 0.01-0.1 wt% based on the weight of the organic nutrient source. These amounts are not limited and are suitably selected depending on the nature or state of wastewater.

The method for treating wastewater using the microbial agent obtained as described above satisfies current wastewater discharge limits. In addition, when the method was applied for 6 months, a daily sludge generation of about 40-50 tons before treatment was reduced by about 85% on average, which corresponds to a decrease in cost of 40-50 million Won (Korean currency). Furthermore, it is expected that an astronomical cost for treatment of sludge will be incurred after the year 2011 from which the ocean dumping of sludge is prohibited.

Hereinafter, the present invention will be described in detail with reference to examples and test examples. It is to be understood, however, that these examples are for illustrative purposes only and are not intended to limit the scope of the present invention.

### Example 1: Culture of microbial mixture

A microorganism-containing soil sample (bamboo humus, pine tree humus, oak waste, or broad-leaved tree humus) was heat-treated at 60 °C for 30 minutes, and then ground finely with a mortar and pestle. Then, 1 g of the sample was taken, suspended in 9 ml of 0.85 % NaCl and diluted by a factor of 10° to 10⁻⁶. 100 *µ*ℓ of each of the diluted suspensions was plated on TSA, BL, and BBL media (DIFCO) and cultured at 28 °C, thereby culturing the microbial mixture.

### Example 2: Preparation of wastewater treatment agent

0.05 kg of the microbial mixture (BM-S-1) cultured as described above, 0.2 kg of powdery chaff, 0.2 kg of powdery peat moss, 2.5 kg of molasses and 0.1 kg of shiitake mushroom waste wood powder were mixed with potable water to a total weight of 100 kg. The mixture was maintained at 15 to 28 °C while air was introduced therein at a rate of 60 cm³/min for 3 days (aeration) and the aeration process was stopped for a period of 3 days. The aeration process and the non-aeration process were repeatedly performed for 30 days, thereby preparing a liquid microbial agent. The total cell count of the liquid microbial agent was 2.8×10⁹ cfu/g.

Meanwhile, 45 kg of crushed chaff and 45 kg of peat moss powder were placed in a shaking incubator and mixed for 30 minutes. 10 kg of the liquid microbial agent was added to the mixed material to obtain a culture medium, and the culture medium was adjusted to a water activity of 50-60%, after which the soil microbial mixture was inoculated onto the culture medium in an amount of 0.02 wt% based on the total weight of the culture medium. The inoculated culture medium was incubated in an incubator at a temperature of 65 to 80 °C for 5 hours while it was rotated at a speed of 120 rpm. The incubated material was cooled to 20 °C and dried in hot air at 60 °C, thereby preparing 90 kg of a powdery microbial agent having a water content of less than 10%.

### Test Example 1: Application to tannery wastewater

The microbial agent prepared in the example of the present invention was applied to a public wastewater treatment system of the Pusan Shinpyung Janglim Leather Industry Association (Korea).

First, 5 kg of a seed, 15 kg of the powdery microbial agent and 25 kg of molasses were placed in a 1-ton tank, and the content of the tank was aged for 24 hours while stirring. 1 ton of the content was diluted in 10 tons of water and activated for 24 hours, after which the dilution was placed in a 30-ton tank.

When the safety inventory of the 30-ton tank was 20 tons, 10 tons of the dilution was added thereto. The liquid microbial agent in the 30-ton tank was controlled at a flow rate of 5 tons per day, and no chemical treatment process was performed. As a result, there was little or no generation of sludge, and the generation of offensive odors was reduced.

As shown in Tables 2 and 3 below (test example for tannery wastewater treatment using the microbial agent), the quality of effluent water was significantly improved as can be seen from the results of measurement of BOD, COD and T-N.

**Table 2**

| | July | | | August | | | September | | | October | | |
|---|---|---|---|---|---|---|---|---|---|---|---|---|
| | COD | SS | T-N | COD | SS | T-N | COD | SS | T-N | COD | SS | T-N |
| Raw water | 2100 | 2500 | 700 | 2200 | 2400 | 680 | 2150 | 2550 | 710 | 2200 | 2400 | 800 |
| Primary settling tank | 250 | 1000 | 240 | 260 | 980 | 231 | 210 | 931 | 210 | 220 | 930 | 210 |
| Secondary settling tank | 90 | 100 | 93 | 88 | 96 | 91 | 82 | 91 | 91 | 91 | 120 | 96 |
| Tertiary settling tank | 15 | 12 | 25 | 18 | 15 | 29 | 23 | 12 | 21 | 10 | 15 | 12 |
| Treatment efficiency (%) ** | 95.7 | 96.0 | 86.7 | 96.0 | 96.0 | 86.6 | 96.2 | 96.4 | 87.2 | 95.9 | 95.0 | 88.0 |
| Treatment efficiency (%)*** | 99.3 | 99.5 | 96.4 | 99.2 | 99.4 | 95.7 | 98.9 | 99.5 | 97.0 | 99.5 | 99.4 | 98.5 |

**Table 3**

| | November | | | December | | |
|---|---|---|---|---|---|---|
| | COD | SS | T-N | COD | SS | T-N |
| Raw water | 2500 | 2600 | 800 | 2400 | 2300 | 650 |
| Primary settling tank | 230 | 1020 | 250 | 270 | 1100 | 270 |
| Secondary settling tank | 96 | 99 | 102 | 110 | 121 | 108 |
| Tertiary settling tank | 27 | 25 | 30 | 45 | 27 | 50 |
| Treatment efficiency (%)** | 96.2 | 96.2 | 87.3 | 95.4 | 94.7 | 83.4 |
| Treatment efficiency (%)*** | 98.9 | 99.0 | 96.3 | 98.1 | 98.8 | 92.3 |

In Tables 2 and 3, unit: mg/; ** treatment efficiency of the secondary settling tank relative to raw water; *** treatment efficiency of the tertiary secondary settling tank relative to raw water.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

### Industrial Applicability

The present invention relates to a method for biologically treating sewage, wastewater, tannery wastewater and livestock manure, and more particularly to a method for biologically treating refractory wastewater, which can biologically treat refractory wastewater without performing physical or chemical pretreatment and can also reduce the generation of various offensive odors and sludge in a wastewater treatment process, and to an agent for treating wastewater.

## Claims

1. A method for biologically treating refractory wastewater, the method comprising the steps of: maintaining a mixture of 0.01-1 wt% of microorganism BM-S-1 (accession number: KCTC 11789BP), 0.1-1 wt% of powdery chaff, 0.1-1 wt% of powdery peat moss, 1-5 wt% of molasses, 0.01-1 wt% of shiitake mushroom waste wood powder and 92-98 wt% of water at 15 to 28 °C to prepare a liquid microbial agent; mixing 50-90 wt% of a medium with 5-50 wt% of the liquid microbial agent to prepare a mixed material; inoculating 100 parts by weight of the mixed material with 0.01-1 part by weight of the microbial mixture (BM-S-1) at a high temperature of 65 °C to 85 °C; incubating the mixed material inoculated at a high temperature; drying the incubated mixed material; incubating the dried mixed material in a liquid state to prepare a microbial solution; and proliferating and activating microorganisms of the microbial solution before introducing the microbial solution into wastewater.

2. The method of claim 1, wherein the wastewater is selected from among livestock manure, tannery wastewater and refractory wastewater.

3. The method of claim 1, wherein the microbial solution is fixed on a carbohydrate medium.

4. A wastewater treatment agent which is prepared by mixing 0.01-1 wt% of microorganism BM-S-1 (accession number: KCTC 11789BP), 0.1-1 wt% of powdery chaff, 0.1-1 wt% of powdery peat moss, 1-5 wt% of molasses, 0.01-1 wt% of shiitake mushroom waste wood powder and 92-98 wt% of water and is used for wastewater treatment.
